# EUROPEAN PATENT SPECIFICATION

(11) **EP 0 380 129 B1**
(45) Date of publication and mention of the grant of the patent: **08.12.1993**
(21) Application number: 90101605.5
(22) Date of filing: 26.01.1990
(51) Int. Cl.: C07C 53/48, C07C 53/50, C07C 51/58

(54) **Process for preparing alpha-bromo-acyl-fluorides**
Verfahren zur Herstellung von alpha-Bromazylfluoriden
Procédé de préparation de fluorures d'alpha-bromoacyles

(30) Priority: 27.01.1989 IT 1920989
(43) Date of publication of application: 01.08.1990
(73) Proprietor: AUSIMONT S.p.A., I-20100 Milano (IT)
(72) Inventor: Desmarteau, Darryl D., Dr., C/o Clemson Univ., S. Carolina 29631 (US); Gregorio, Guglielmo, Dr., I-20161 Milan (IT); Navarrini, Walter, Dr., I-20010 Boffalora Ticino, Milan (IT)
(74) Representative: Barz, Peter, Dr.

(56) References cited:
- GB-A- 363 099
- US-A- 3 321 515

## Description

The present invention is concerned with the preparation of α-bromo-acyl fluorides of formula

R¹-CFBr-COF

wherein R¹ is a fluorine atom or a perfluoroalkyl radical R_{F} containing from 1 to 10 carbon atoms.

These compounds are generally disclosed in EP-A-194 862 and are prepared according to the process of IT-A-1 134 028.
IT-A-1 134 028 describes a process for preparing CF₃COCl by reacting CF₃-CCl₃ with SO₃ using a Hg catalyst. The CF₃ COCl obtained may be reacted with HF to produce CF₃COF.

It has been found, according to the present invention, that the above α-bromo-acyl fluorides can be prepared in good yield and with high selectivity by reacting the corresponding perfluoroepoxides 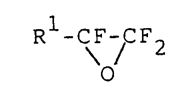
with a trialkyl-silyl bromide in the presence of activated charcoal.

One object of the present invention thus is the provision of a process for the preparation of the above α-bromo-acyl fluorides, which makes it possible to obtain the desired products in good yield and with high selectivity.

This object is achieved by means of the process according to the present invention for preparing α-bromo-acyl fluorides of formula

R¹-CFBr-COF

wherein:
R¹ is a fluorine atom or a perfluoroalkyl radical R_{F} containing from 1 to 10 carbon atoms.

This process is characterized in that a perfluoroepoxide of formula 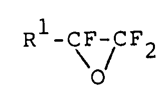
is reacted, in the presence of activated charcoal, with a trialkyl-silyl bromide of formula 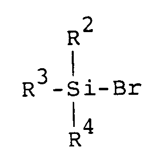
wherein R², R³ and R⁴, the same or different from each other, are alkyl groups containing from 1 to 8 carbon atoms.

The above reaction may be represented as follows: 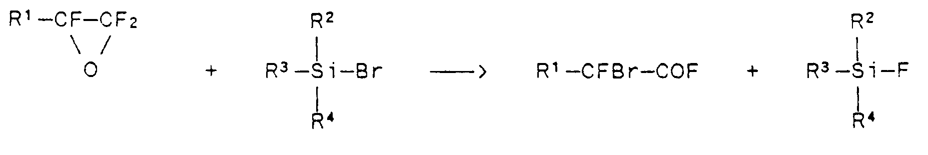
The reaction temperature generally is within the range of from -100° to +150°C. The reaction is preferably carried out under a pressure between atmospheric pressure and 5 atm.

The molar ratio of trialkyl-silyl bromide to perfluoroepoxide generally ranges from 0.5 to 2. Preferably, said ratio is from 0.9 to 1.1.

As far as the amount of activated charcoal is concerned, good results may be obtained, for instance, with a weight ratio of activated charcoal to epoxide (calculated as hexafluoropropene oxide) within the range of from 0.1 to 100. The reaction can be carried out in a solvent medium, in particular in a chlorofluorocarbon solvent such as, e.g., CFCl₃, CF₂Cl₂ or CF₃-CF₂Cl.

In the perfluoroepoxide used as the starting compound, the R¹ group preferably is a fluorine atom or a perfluoroalkyl radical R_{F} of from 1 to 5 carbon atoms. The most preferred epoxides are: 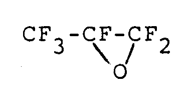
and 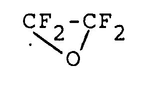
In the trialkyl-silyl bromide used as the starting material, R², R³ and R⁴ preferably are -CH₃, -C₂H₅ or -C₃H₇ groups. The three groups R², R³ and R⁴ usually are identical.

The α-bromo-acyl fluorides obtainable by means of the process according to the present invention are highly valuable intermediates. By fluorination, they can be converted into the corresponding fluoro-oxy-compounds of formula

R¹-CFBr-CF₂OF

which can react with CFCl=CFCl to yield ethers of formula

R¹-CFBr-CF₂O-CFCl-CF₂Cl

which, by de-chlorination, yield fluoro-bromo-alkyl-vinyl ethers of formula

R¹-CFBr-CF₂O-CF=CF₂

The latter compounds are suitable for use as comonomers in polymerization reactions with fluorinated monomers. In the copolymer obtained, the bromine atoms may serve as sites for crosslinking.

The fluorination of the α-bromo-acyl fluoride can be carried out, e.g., according to the process of EP-A-194,862, and the reaction of the fluorooxy-compound obtained with CFCl=CFCl can be carried out, e.g., according to the process disclosed in EP-A-201,871.

When the present invention is practiced at an industrial level, a gaseous stream of both reactants often is advantageously passed over a layer of activated charcoal.

The following examples are given for illustrative purposes only.

### Example 1

A reactor of 250 ml capacity was charged with 3.2 g of activated charcoal. The reactor was cooled down to -196°C and 5 mmol of 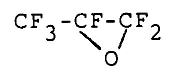
were introduced. Thereafter, 5.2 mmol of (C₂H₅)₃SiBr were added. The reactor was allowed to reach room temperature within 14 hours wehreafter it was cooled down to -196°C and its content was distilled under vacuum through a line containing traps kept at -60°C and -196°C. (C₂H₅)₃SiF was collected inside the trap at -60°C, and in the trap at -196°C 4.7 mmol of CF₃CFBrCOF were collected. This corresponds to a yield of 94%, based on hexafluoropropene oxide.

### Example 2

A gas containing tetrafluoroethylene epoxide was bubbled, at a rate of 10 l/h, into 50 ml of trichlorofluoromethane cooled at -78°C and containing 12.5 g of (CH₃)₃SiBr and 15 g of charcoal powder previously dried at 400°C in a N₂ stream. The gas composition was: 16% C₂F₄O; 59% C₂F₄; 14% COF₂; 11% O₂.

The main part of C₂F₄O and C₂F₄ condensed in the liquid phase while most of the O₂ and COF₂ escaped with the gases.

After 2 hrs the stream was interrupted and the mixture was stirred at -78°C for additional 20 hrs. The slurry was then warmed to 0°C and degassed.

A sample of the slurry, diluted in carbon tetrachloride, was analysed by NMR, revealing the presence of CF₂BrCOF beside some CF₂BrCOBr (¹⁹F NMR signals at +64 ppm and +60 ppm referred to CFCl₃).

By distillation 6.5 g of CF₂BrCOF (yield 57% referred to C₂F₄O) were recovered.

## Claims

1. Process for preparing α-bromo-acyl fluorides of formula
R¹-CFBr-COF
wherein:
R¹ is a fluorine atom or a perfluoroalkyl radical R_{F} containing from 1 to 10 carbon atoms, characterized in that a perfluoroepoxide of formula 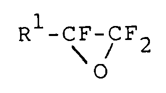 is reacted, in the presence of activated charcoal, with a trialkyl-silyl bromide of formula 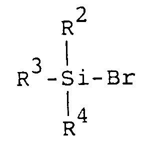 wherein R², R³ and R⁴, the same or different from each other, are alkyl groups containing from 1 to 8 carbon atoms.

2. Process according to claim 1, characterized in that the reaction is carried out at a temperature of from -100° to +150°C.

3. Process according to any one of claims 1 and 2, characterized in that in the perfluoroepoxide used as the starting compound R¹ is a fluorine atom or a perfluoroalkyl radical R_{F} of from 1 to 5 carbon atoms.

4. Process according to claim 3, characterized in that in the perfluoroepoxide used as the starting compound R¹ is a fluorine atom or a -CF₃ radical.

5. Process according to one or more of the preceding claims, characterized in that in the trialkyl-silyl bromide used as the starting compound the radicals R², R³ and R⁴ are -CH₃, -C₂H₅ or -C₃H₇ groups.

## Patentansprüche

1. Verfahren zur Herstellung von α-Bromacylfluoriden der Formel
R¹-CFBr-COF
worin:
R¹ ein Fluoratom oder ein Perfluoralkylrest R_{F}, der 1 bis 10 Kohlenstoffatome enthält, ist, dadurch gekennzeichnet daß ein Perfluorepoxid der Formel 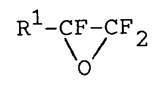 in Anwesenheit von aktivierter Holzkohle mit einem Trialkylsilylbromid der Formel 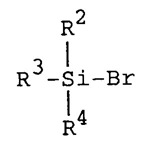 worin R², R³ und R⁴, gleich oder verschieden voneinander, Alkylgruppen, die 1 bis 8 Kohlenstoffatome enthalten, darstellen, umgesetzt wird.

2. Verfahren nach Anspruch 1, dadurch gekennzeichnet, daß die Umsetzung bei einer Temperatur von -100 bis +150°C durchgeführt wird.

3. Verfahren nach irgendeinem der Ansprüche 1 und 2, dadurch gekennzeichnet, daß in dem als Ansgangsverbindung eingesetzten Perfluorepoxid R¹ ein Fluoratom oder ein Perfluoralkylrest R_{F} mit 1 bis 5 Kohlenstoffatomen ist.

4. Verfahren nach Anspruch 3, dadurch gekennzeichnet, daß in dem als Ausgangsverbindung eingesetzten Perfluorepoxid R¹ ein Fluoratom oder ein CF₃-Rest ist.

5. Verfahren nach irgendeinem der vorangehenden Ansprüche, dadurch gekennzeichnet, daß in dem als Ausgangsverbindung eingesetzten Trialkylsilylbromid die Reste R², R³ und R⁴ Gruppen -CH₃, -C₂H₅ oder -C₃H₇ sind.

## Revendications

1. Procédé de préparation des fluorures d'alpha-bromoacyles de formule:
R¹CFBr-COF
dans laquelle:
R¹ représente un atome de fluor ou un radical perfluoroalkyle R_{F} contenant entre 1 et 10 atomes de carbone,
caractérisé en ce qu'un perfluoroépoxyde de formule: 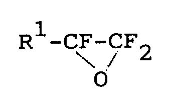 réagit, en présence de charbon de bois activé, avec un bromure de trialkyl-silyle de formule: 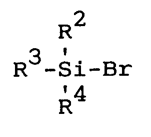 dans laquelle:
R², R³ et R⁴ identiques ou différents les uns des autres représentent des radicaux alkyles contenant entre 1 et 8 atomes de carbone.

2. Un procédé selon la revendication 1, caractérisé en ce que la réaction est réalisée à une température comprise entre -100°C et +150°C.

3. Un procédé selon l'une quelconque des revendications 1 et 2, caractérisé en ce que dans le perfluoroépoxyde utilisé comme dérivé de départ R¹ représente un atome de fluor ou un radical perfluoroalkyle R_{F} contenant entre 1 et 5 atomes de carbone.

4. Un procédé selon la revendication 3, caractérisé en ce que dans le perfluoroépoxyde utilisé comme dérivé de départ R¹ représente un atome de fluor ou un radical -CF₃.

5. Un procédé selon l'une ou plusieurs des revendications précédentes, caractérisé en ce que dans le bromure de trialkyl-silyle utilisé comme dérivé de départ les radicaux R², R³ et R⁴ sont des radicaux -CH₃, -C₂H₅ ou -C₃H₇.
